# EUROPEAN PATENT APPLICATION

(11) **EP 0 971 231 A1**
(43) Date of publication of application: **12.01.2000**
(21) Application number: 98905747.6
(22) Date of filing: 03.03.1998
(51) Int. Cl.: G01N 33/48, A61B 5/14

(54) **BLOOD COLLECTING TUBE AND METHOD OF ISOLATION OF SERUM AND BLOOD PLASMA USING THE TUBE**

(30) Priority: 03.03.1997 JP 8861797
(71) Applicant: Kabushiki Kaisha IR Medical, Tokyo 134-0088 (JP)
(72) Inventor: TASHIRO, Katushi, Imba-gun, Chiba-ken 286-0201 (JP); SUZUKI, Issei, Yamato-shi, Kanagawa-ken 242-0011 (JP)
(74) Representative: Goddar, Heinz J., Dr.
(86) International application number: JP9800881
(87) International publication number: WO9839650

(57) **Abstract**

The present invention provides a blood collecting tube comprising a cylindrical vessel body with bottom, a cylindrical body which is telescopically inserted into the vessel body from an opening end of the vessel body and both ends of which are opening, and a stopper which is bonded to or engaged with the upper end opening of the cylindrical body integrally with the cylindrical body and in a liquid sealed state, whereby after collecting blood the blood is subjected to centrifugal separation, the stopper assembly of the blood collecting tube is withdrawn and thus it is possible to take out the blood serum and plasma alone in the tube.

## Description

### FIELD OF THE INVENTION

The present invention relates to a blood collecting tube which is used for storing the blood collected from a blood testee or patient and taking blood serum and/or blood plasma of the blood thus collected, and a process for separating the blood serum and/or the blood plasma by utilizing the tube.

### BACKGROUND OF THE INVENTION

Conventionally the taking-out of blood serum and/or blood plasma from a blood collecting tube has been carried out in such a manner that the blood serum and/or blood plasma are collected bypipet or the blood collecting tube itself containing the collected blood is inverted thereby to transfer into another test tube the blood serum and plasma alone to be the supernatant liquid on the gel.

Thus to transfer the blood serum and plasma from a blood collecting tube into another test tube by using the pipet or reverting the blood collecting tube it is necessary to use a separate vessel like test tube. Further, after the transfer of the sample into the separate vessel it is required to fix an identification barcode to said vessel or to mark the patient 's name. This makes it costly to manufacture the tools and causes complicated working.

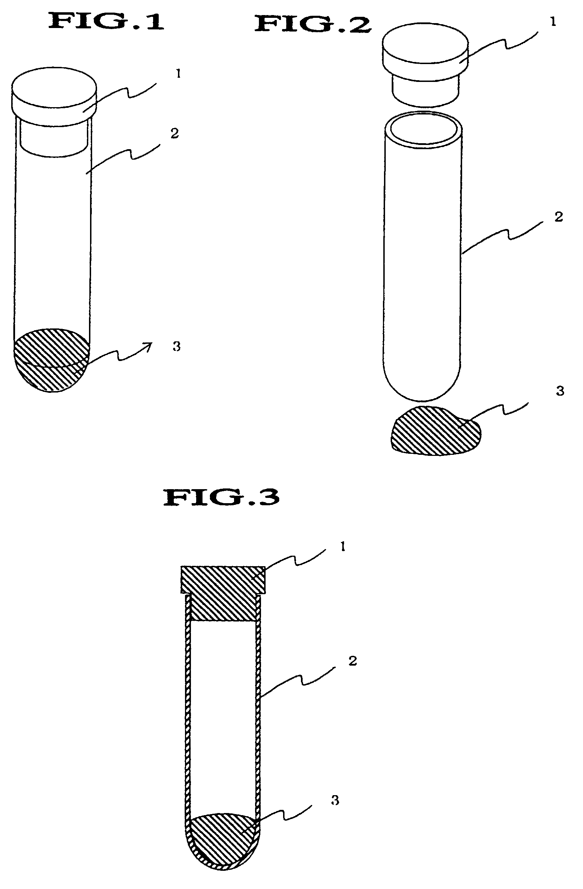

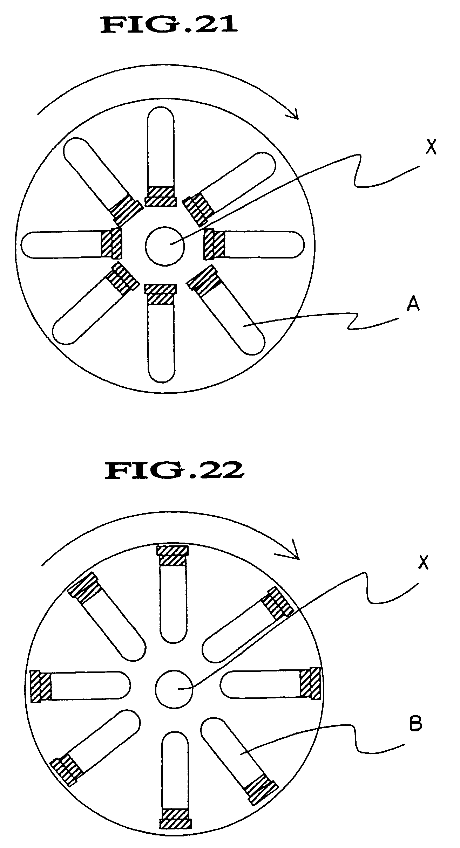

On the other hand, there has conventionally been proposed even a blood collecting tube containing gel as shown in FIGs. 1 to 3. In FIGs. 1 to 3 the reference numeral 1 designates a stopper, 2 a blood collecting tube body, and 3 a gel for separating the corpuscle components from the blood serum and/or the blood plasma. Such conventional blood collecting tubes A containing a gel are disposed, as illustrated in FIG. 21, with the stoppers 1 directed toward the axial center X of a centrifugal separator, and they are subject to centrifuging so as to separate the blood serum and/or plasma from the corpuscle components.

The first object of the present invention is therefore to provide a blood collecting tube where said problem is solved and which is usable for directly taking out of it the blood serum and/or the blood plasma.

The second object of the present invention is to provide a process for separating the blood serum and/or plasma from other blood components by utilizing said blood collecting tube.

### DISCLOSURE OF THE INVENTION

To achieve said first object, according to one aspect of the present invention, there is provided a blood collecting tube comprising: a cylindrical vessel body with bottom; a cylindrical body which is telescopically inserted in to the vessel body from the opening end of the vessel body and both the ends of which are opening; and a stopper which is bonded to or inserted into the upper end opening of the cylindrical body integrally with the cylindrical body and in a liquid sealed state.

A gel for separating the blood serum and/or plasma from the corpuscle components is put at the bottom inside the cylindrical container body with bottom.

The cylindrical body is constructed in such a manner that when it is inserted into the cylindrical container body with bottom it has a sufficient length to be able to reach the gel put at the bottom of the cylindrical container body with bottom.

The cylindrical body and the stopper are formed integrally by a synthetic resinous material. The bottom end of the cylindrical body may be tapered.

According to another aspect of the present invention, to achieve the second object there is provided a method of separating blood serum and/or plasma, characterized in that a great number of blood collecting tubes containing blood according to the first invention are prepared, they are radially arranged with their stoppers directed in the outer periphery about the bottoms of the cylindrical vessel bodies with bottom of the respective blood collecting tubes, and the blood serum and/or plasma are separated by means of centrifuging from the blood in each blood collecting tube.

The blood collecting tubes containing the blood now collected by using said tubes with gel are arranged in a centrifugal separator, with their stoppers directed downward. Such arrangement of the blood collecting tubes is contrary to the conventional manner for arrangement in a centrifugal separator. In case the centrifuging is applied to the blood collecting tubes in such state, it is adapted that the bottoms of the tubes are positioned near the center of the separator while the stoppers of the tubes are positioned away from the center from the bottoms.

In the blood collecting tubes thus finished for centrifugal separation, the liquid is separated in three layers of a corpuscle portion, a gel portion and a blood serum and plasma portion by the gravity applied by the centrifugal force.

To add, the gel acts such that when the blood is centrifugally separated the boundary surface between the corpuscle portion and the blood serum and plasma portion is retained for separation so as not to be mixed.

The blood collecting tubes are perpendicularly set at a tube stand with their bottoms being downwardly directed in the conventional manner. Then the blood serum and plasma portion alone of the blood thus separated in three layers falls down from the cylindrical body whereby the remaining corpuscle portion remains fixed to the upper portion i.e. the stopper portions by the retaining force of the physical strength possessed by the gel.

It is therefore necessary that the end of each cylindrical body has a sufficient length to secure the capacity of accommodating the corpuscle portion and the gel portion.

### BRIEF DESCRIPTION OF THE DRAWINGS

FIG. 1 is a perspective view showing a conventional blood collecting tube containing gel;
FIG. 2 is a perspective view in which the structural elements of the blood collecting tube with gel of FIG. 1 are disassembled;
FIG. 3 is a sectional view showing the blood collecting tube with gel of FIG. 1;
FIG. 4 is a perspective view showing the stopper and the cylindrical body according to one embodiment of the invention;
FIG. 5 is a perspective view showing the structural elements of FIG. 4 assembled in one body;
FIG. 6 is a perspective view showing the state where the structural elements of FIG. 5 are reverted;
FIG. 7 is a disassembled perspective view showing the blood collecting tube according to one embodiment of the invention;
FIG. 8 is a sectional view showing each of the structural elements of FIG. 7;
FIG. 9 is a perspective view showing the blood collecting tube of FIG. 7, in the assembled state;
FIG. 10 is an enlarged sectional view of the blood collecting tube shown in FIG. 9;
FIG. 11 is a sectional view showing the blood collecting tube according to another embodiment of the invention;
FIG. 12 is a disassembled sectional view showing still another embodiment of the invention;
FIG. 13 is a sectional view showing the blood collecting tube of FIG. 12 in the assembled state;
FIG. 14 is a perspective view showing the state where blood is collected in the blood collecting tube of the invention shown in FIGs. 4 to 10;
FIG. 15 is a perspective view showing the state of the blood collecting tube containing the blood, shown in FIG. 14, has been reverted vertically so as to be subjected to centrifugal separation;
FIG. 16 is a perspective view showing the state after the blood collecting tube of FIG. 15 after centrifugal separation has been erected in a test tube stand;
FIG. 17 is a perspective view showing the state where the stopper and the cylindrical body have been dismantled from the blood collecting tube of FIG. 16;
FIG. 18 is a perspective view showing the structural elements of the blood collecting tube according to still another embodiment of the invention;
FIG. 19 is a disassembled sectional view showing the blood collecting tube having the structural elements of FIG. 18;
FIG. 20 is a sectional view where the blood collecting tube of FIG. 19 is illustrated in the assembled state;
FIG. 21 is a schematic diagram in which a centrifugal separator installed with the blood collecting tubes according to known process is observed from the top;
FIG. 22 is a schematic diagram in which a centrifugal separator installed with the blood collecting tubes according to the present invention is observed from the top; and
FIG. 23 is a disassembled perspective view showing the structural elements of the blood collecting tube according to another embodiment of the invention.

### THE BEST MODE FOR CARRYING OUT THE INVENTION

The present invention will now be described, by way of embodiments, with reference to the accompanying drawings.

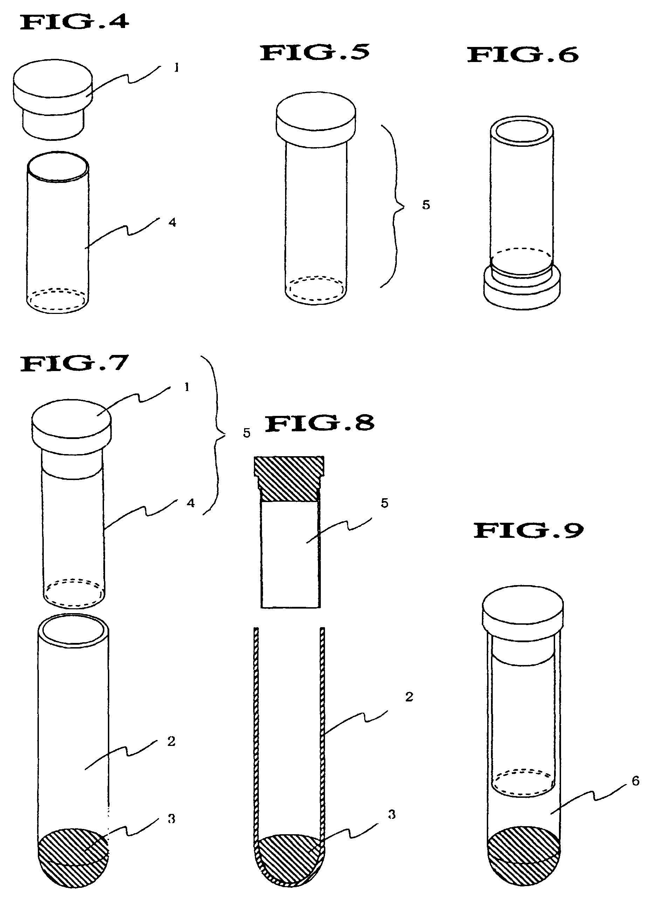

FIGs. 4 to 10 show one embodiment of the present invention. The blood collecting tube illustrated comprises a stopper or plug 1, a cylindrical vessel body 2 having internally a bottom for putting a gel 3 to separate the blood serum and/or the blood plasma from the corpuscle components, and a cylindrical body 4 the both ends of which are opening. The cylindrical body 4 has an outside diameter for close contact with the inside diameter of the cylindrical vessel body 2 and is telescopically fitted from the opening end of the body 2. As illustrated in FIGs. 8 and 9, the stopper 1 is bonded to or fitted into the upper end opening of the cylindrical body 4 integrally with the body 4 in a liquid sealed state thereby forming a stopper assembly 5. Preferably, each of the structural elements is made of a suitable synthetic material, and the stopper 1 is constituted by a rubber-like elastic material.

Then, the invention will be described in respect of the separation of the blood serum and plasma components by the use of the blood collecting tube thus constructed and illustrated.

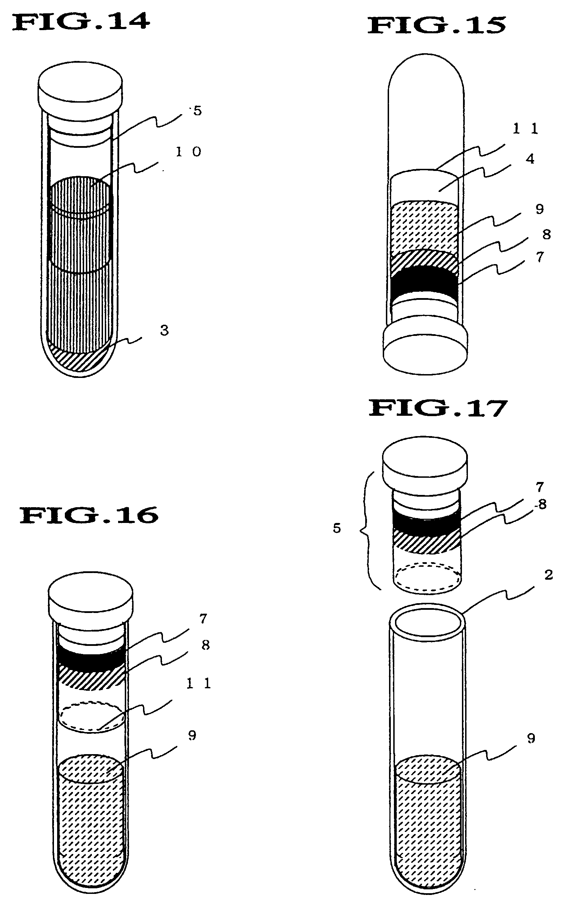

Firstly, as illustrated in FIG. 14, blood is collected from the patient in the ordinary manner by using the blood collecting tube. Secondly, as illustrated in FIG. 15, the blood collecting tube B containing blood is set so that the stopper assembly 5 may be directed downwardly. Then, as shown in FIG. 22, the respective blood collecting tubes 1 are disposed with the bottoms of said cylindrical vessel bodies 4 with bottom while surrounding the axial center X, axially with the stoppers 1 directed externally and peripherally thereby to separate the blood serum and plasma from the blood of the respective blood collecting tubes by means of centrifugal separator.

The blood 10 in the blood collecting tubes, which has thus been centrifugally separated, is separated, as shown in FIG. 15, into the three layers of a corpuscle portion 7, a gel portion 8 and a blood serum and plasma portion 9.

The blood collecting tube after completion of the centrifugal separation (see FIG. 15) is erected on the test tube stand (not shown) in the conventional manner with the bottom of the tube directed downwardly (see FIG. 16).

At that time, regarding the blood within the blood collecting tube, which is separated in the three layers, the blood serum and plasma portion only falls down by erecting the blood collecting tube with its bottom directed downwardly so as to be collected at the bottom of said tube as shown in FIGs. 16 and 17.

On the other hand, the corpuscle component retained by the gel 8 in the upper portion in the stopper assembly 5 of the test tube is maintained by the physical strength of the gel 8 without falling down.

In that state, as illustrated in FIG. 17, the stopper assembly 5 of the blood collecting tube is withdrawn from the container body 2 whereby the stopper assembly 5 is to be withdrawn from the container body 2 while the corpuscle component 7 is being maintained by the gel within the cylindrical body 4.

Thus, only the blood serum and plasma are to remain in the container body 2 of the blood collecting tube, so that only the blood serum and plasma can be easily taken out, followed by easily treatment of the blood thereafter.

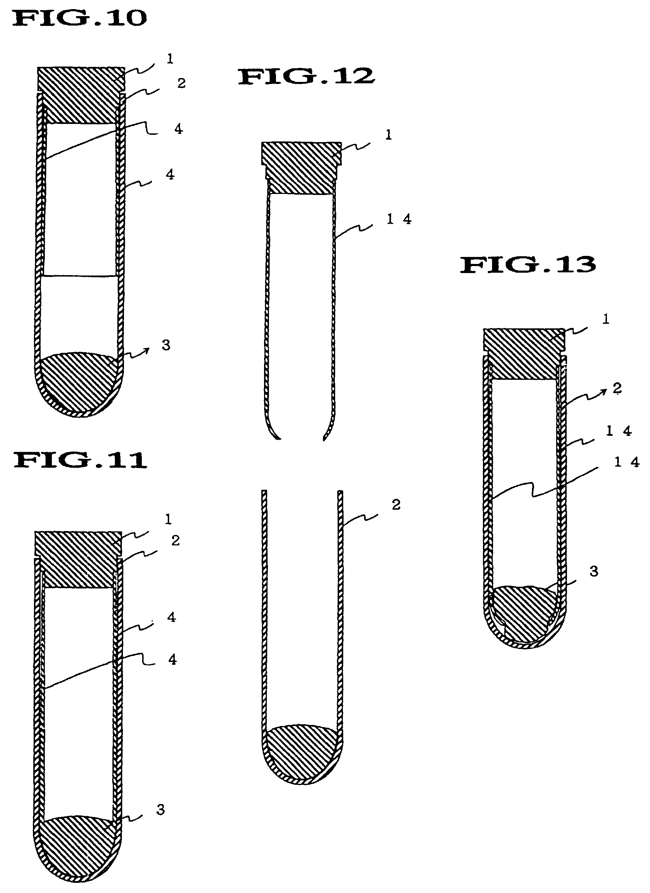

FIG. 11 shows a second embodiment of the present invention, in which case the cylindrical body 4 is elongated to reach the position of the gel 3 put at the bottom of the container body 2.

FIGs. 12 and 14 shows a third embodiment of the invention, in which the cylindrical body 14 is elongated like in FIG. 11 and its end is tapered as shown.

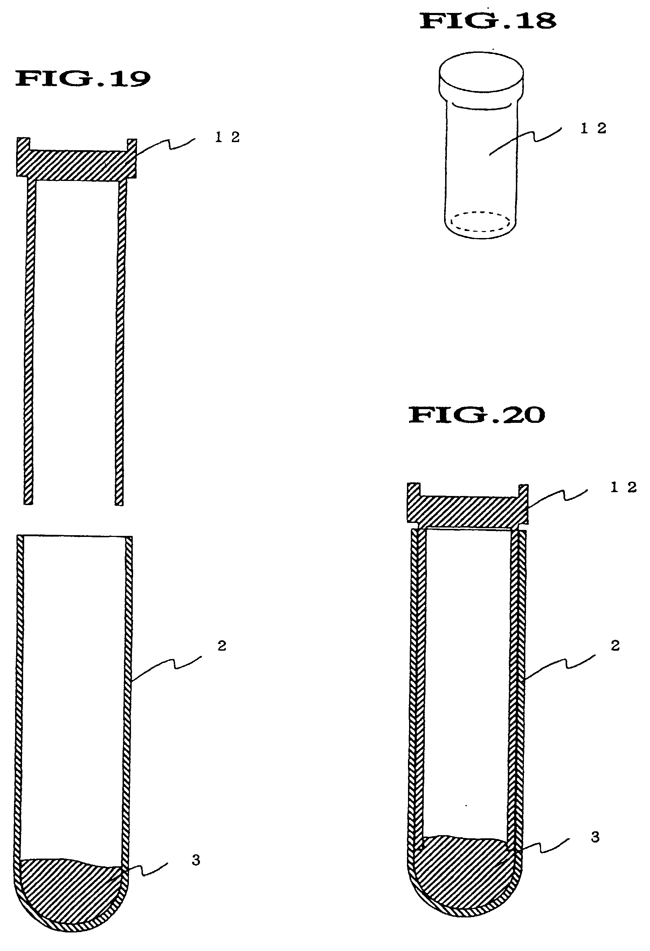

A fourth embodiment of the invention is shown in FIGs. 18 to 20. In this embodiment the stopper and the cylindrical body are formed integrally as an element 12, and one end of this one body element 12 is elongated so as to reach the gel 3 contained at the bottom of said vessel body 2 as illustrated in FIG. 20.

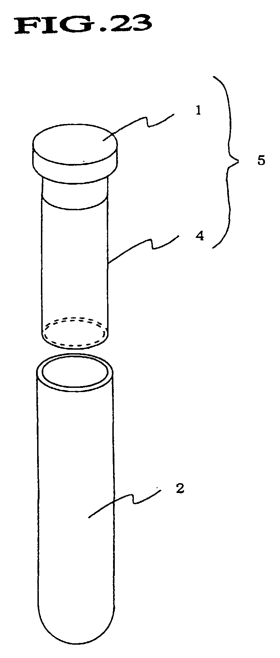

A fifth embodiment of the invention is shown in FIG. 23, which embodiment being substantially the same as the structure of the first embodiment of FIGs. 4 to 10, excepting that the gel is not put at the bottom of the vessel body 2.

### INDUSTRIAL FIELD OF THE INVENTION

As described above in detail, the blood collecting tube according to the present invention has the merits that after collecting blood the blood is subjected to centrifugal separation, the tube is erected in a test tube stand, and after lapse of a predetermined period of time the stopper assembly of the blood collecting tube is withdrawn whereby it is possible to take out the blood serum and plasma alone in the tube which has now collected the blood, without any specific operation. According to the present invention, therefore, it is no longer necessary to take out the blood serum and/or the blood plasma by a pipet or to transfer into a test tube the blood serum and/or blood plasma alone to be the supernatant liquid on the gel by upturning the blood collecting tube containing the collected blood, which has so far been operated. As a result, the undermentioned industrial merits will be obtained.
(a) There are no longer needed the test tube itself for the transfer of the blood serum and plasma and the operation for such transfer.
(b) Since it is capable of taking only the blood serum and/or the blood plasma with the blood collecting tube used for collecting blood it is no longer required to mark the patient's name or identification number for identifying the patient's blood serum or plasma, which has so far been needed when the blood has been transferred into a test tube.
(c) There is no longer possibility of contagion to the operator, which is involved in the transfer.
(d) If the blood collecting tube of the present invention is used in various machines with which only the blood serum and plasma are automatically taken out from the blood in the blood collecting tube, it is possible to reduce some of the automation steps.

## Claims

1. A blood collecting tube comprising: a cylindrical vessel body with bottom; a cylindrical body which is telescopically inserted into the vessel body from an opening end of the vessel body and both ends of which are opening; and a stopper which is bonded to or engaged with the upper end opening of the cylindrical body integrally with the cylindrical body and in a liquid sealed state.

2. A blood collecting tube as claimed in claim 1, wherein a gel for separating the blood serum and/or plasma from the corpuscle components is put at the bottom inside the cylindrical vessel body.

3. A blood collecting tube as claimed in claim 2, wherein the cylindrical body is constructed in such a manner that when it is inserted into the cylindrical container body it has a sufficient length to be able to reach the gel put at the bottom of the cylindrical container body.

4. A blood collecting tube as claimed in any one of claims 1 to 3, wherein the cylindrical body and the stopper are formed integrally by a synthetic resinous material.

5. A blood collecting tube as claimed in any one of claims 1 to 4, wherein the bottom end of the cylindrical body may be tapered.

6. A method of separating blood serum and/or plasma by using a blood collecting tube as claimed in any one of claims 1 to 5, wherein a number of blood collecting tubes containing blood are prepared, the blood collecting tubes are radially arranged with their stoppers directed in the outer periphery about the bottoms of the cylindrical vessel bodies with bottom of the respective blood collecting tubes, and the blood serum and/or plasma are separated by means of centrifuging from the blood in each blood collecting tube.
